Europäisches Patentamt

European Patent Office

Office européen des brevets

(19)

(11) **EP 1 699 014 A2**

(12) **EUROPEAN PATENT APPLICATION**

(43) Date of publication:
06.09.2006 Bulletin 2006/36

(51) Int Cl.:
*G06T 5/00* (2006.01)      *G06T 11/00* (2006.01)
*A61B 6/00* (2006.01)

(21) Application number: 05018698.0

(22) Date of filing: 29.08.2005

(84) Designated Contracting States:
**AT BE BG CH CY CZ DE DK EE ES FI FR GB GR
HU IE IS IT LI LT LU LV MC NL PL PT RO SE SI
SK TR**
Designated Extension States:
**AL BA HR MK YU**

(30) Priority: **04.03.2005 US 658210 P
31.03.2005 US 94468**

(71) Applicants:
• **KABUSHIKI KAISHA TOSHIBA**
**Tokyo 105-8001 (JP)**

• **Toshiba Medical Systems Corporation
Otawara-shi,
Tochigi-ken 324-8550 (JP)**

(72) Inventor: **Taguchi, Katsuyuki
Intellectual Property Division
Otawara-shi
Tochigi 324-8550 (JP)**

(74) Representative: **Kramer - Barske - Schmidtchen
Radeckestrasse 43
81245 München (DE)**

(54) **Volumetric computed tomography system for imaging**

(57)     A method for reconstructing an image, including obtaining (1401) projection data using an X-ray detector and one of a cone-beam X-ray generator and a fan-beam X-ray generator, filtering (1402) the obtained projection data using a ramp-based filtering function to generate filtered projection data, weighting (1402) the filtered projection data to compensate for redundant projection data, and reconstructing (1403) the image by back-projecting the weighted projection data along a radial path.

FIG. 14

**Description**

The present invention is generally directed to a method of imaging. More specifically, the present invention is directed to the generation of images from angularly disconnected computed tomography projection data sets. The present invention is also directed to the correction of artifacts arising in cardiac imaging.

The present invention includes the use of various technologies referenced and described in the documents identified in the following LIST OF REFERENCES, which are cited throughout the specification by the corresponding reference number in brackets:

LIST OF REFERENCES

[1] D. L. Parker, "Optimal short scan convolution reconstruction for fan beam CT," Medical Physics, vol. 9, pp. 254-257, 1982.
[2] K. Sourbelle and W. A. Kalender, "Generalization of Feldkamp reconstruction for clinical spiral cone-beam CT," Proceedings of fully 3D conference, 2003.
[3] F. Noo, M. Defrise, R. Clackdoyle, and H. Kudo, "Image reconstruction from fan-beam projections on less than a short scan," Physics in Medicine and Biology, vol. 47, pp. 2525-2546, 2002.
[4] R. Manzke, M. Grass, T. Kohler, and R. Proksa, "Extended cardiac reconstruction (ECR): A helical cardiac cone beam reconstruction method," Proceedings of fully 3D conference, 2003.
[5] R. Manzke, M. Grass, T. Nielsen, G. Shechter, and D. Hawkes, "Adaptive temporal resolution optimization in helical cardiac cone beam CT reconstruction," Medical Physics, vol. 30, pp. 3072-3080, 2003.
[6] M. Kachelriess, S. Ulzheimer, and W. A. Kalender, "ECG-correlated image reconstruction from subsecond multi-slice spiral CT scans of the heart," Medical Physics, vol. 27, pp. 1881-1902, 2000.
[7] H. Anno, "Development of cardiac imaging methods employing the multislice computed tomographic system Aquilion with scanning at 0.4 second/rotation," Medical Review, Toshiba Medical Systems Corporation, March 2002, available on the internet at www.toshiba-medical.co.jp/tmp/english/review.
[8] T. Ota and M. Hiraoka, "X-ray Computed Tomography", Japanese Patent Publication No. 2002-011001.
[9] A. Zamyatin, K. Taguchi, and M. Silver, "Practical hybrid convolution algorithm for CT reconstruction," IEEE Nuclear Science Symposium and Medical Imaging Conference, Rome, Italy, 2004.
[10] K. Taguchi, Computed Tomography System and Method, U.S. Patent No. 6,466,640.
[11] K. Taguchi and H. Anno, "High temporal resolution for multi-slice helical computed tomography," Medical Physics, vol. 27, pp. 861-872, 2000.
[12] H. Hu, T. Pan, and Y. Shen, "Multi-slice helical CT: Image temporal resolution," IEEE Transactions on Medical Imaging, vol. 19, pp. 384-390, 2000.
[13] B. Ohnesorge, T. Flohr, C. Becker, A. F. Kopp, U. J. Schoepf, U. Baum, A. Knez, K. Klingenbeck-Regn, and M. F. Reiser "Cardiac imaging by means of electrocardiographically gated multisection spiral CT: Initial experience," Radiology, vol. 217, pp. 564-571, 2000.
[14] H. Anno, "The usefulness of the 0.4 sec scan MSCT: Cardiac application," Innervision, May 2003.
[15] T. Flohr and B. Ohnesorge, "Heart rate optimization of spatial and temporal resolution for ECG gated multislice spiral CT of the heart," Journal of Computer Assisted Tomography, vol. 25, pp. 907-923, 2001.
[16] T. Flohr, U.J. Schoepf, A. Kuettner, S. Halliburton, H. Bruder, C. Suess, B. Schmidt, L. Hoffmann, E. K. Yucel, S. Schaller, and B. M. Ohnesorge, "Advances in cardiac imaging with 16-section CT systems," Academic Radiology, vol. 10, pp. 386-401, 2003.
[17] T. Flohr, B. Ohnesorge, H. Bruder, K. Stiersdorfer, J. Simon, C. Suess, and S. Schaller, "Image reconstruction and performance evaluation for ECG gated spiral scanning with a 16-slice CT system," Medical Physics, vol. 30, pp. 2650-2662, 2003.
[18] M. Grass, R. Manzke, T. Nielsen, P. Koken, R. Proksa, M. Natanon, and G. Shechter, "Helical cardiac cone beam reconstruction using retrospective ECG gating," Physics in Medicine and Biology, vol. 48, pp. 3069-3084, 2003.
[19] K. Saito, "ECG gated x-ray CT apparatus," Japanese Patent disclosure JS63-242,239, October 7, 1988.
[20] J. Hsieh, T.-S. Pan, Y. Shen, S. J. Woloschek, M. E. Woodford, and K. C. Acharya, "Methods and apparatus for cardiac imaging with conventional tomography," U.S. Patent No. 6,639,965 B1, October 28, 2003.
[21] C. R. Crawford and K. F. King, "Computed tomography scanning with simultaneous patient translation," Medical Physics, vol. 17, pp. 967-982, 1990.
[22] K. Taguchi, B. S. Chiang, and M. D. Silver, "A new weighting scheme for cone-beam helical CT to reduce the image noise," Physics in Medicine and Biology, Vol. 49, pp. 2351-2364, 2004.
[23] Y. Liu, H. Liu, Y. Wang, and G. Wang, Halfscan cone-beam CT fluoroscopy with multiple x-ray sources, Medical Physics, vol. 28, pp. 1466-1471.

The entire contents of each reference listed in the above LIST OF REFERENCES are incorporated herein by reference.

Considerable progress has been made in the field of cardiac imaging. However, several key problems remain unaddressed by conventional techniques. In particular, two general observations can be made regarding the shortcomings of conventional cardiac imaging techniques and algorithms.

The first general observation one can make is that conventional image reconstruction algorithms do not allow one to (1) use data which is not parallel but divergent in the xy plane (e.g., data acquired using fan-beam or cone-beam scanning); (2) use a filtered backprojection algorithm based on ramp-filtering; and (3) use disconnected projection data, i.e., data associated with disconnected cyclic projection angles.

A halfscan technique based on a weighted fan-beam ramp-filtering algorithm with connected projection data has been discussed [1]. The projection data in this technique are connected over an angular range of $\pi+2\gamma_m$, wherein $\pi$ represents a half rotation and $\gamma_m$ represents the half of the fan angle. This technique uses weighting to compensate for redundant data samples prior to using ramp-filtering and fan-beam backprojection. However, it fails to allow one to use disconnected projection data.

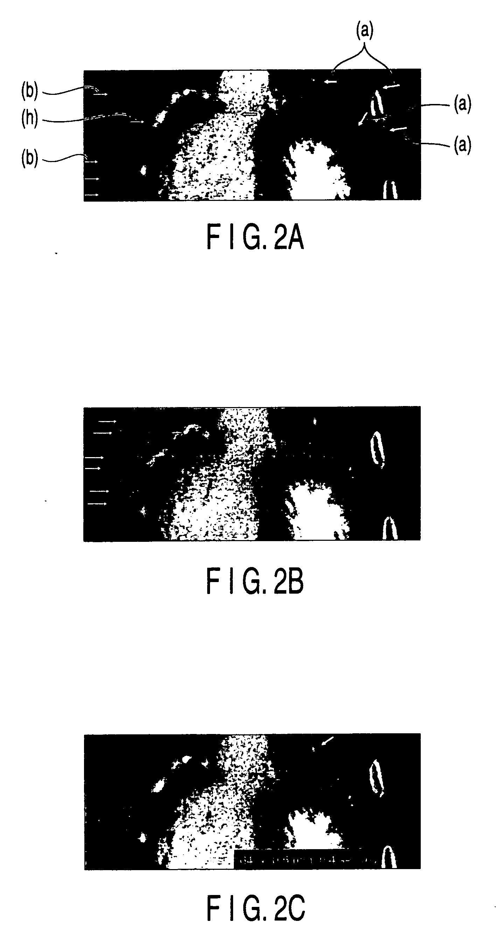

Dividing the projection angle over the halfscan region into a plurality of "patches" (or "segments") separated in time has been discussed [2]. The patches correspond to the same cardiac phase and are acquired at different times during an entire helical scan. An ECG signal, recorded in parallel with the helical scan, can be used to re-sort (or re-bin) projection data from different rotations to form a connected cone-beam projection data set. However, the projection data "reconnected" therein are connected over the cyclic projection angle $\beta'$, wherein $\beta'=\mathrm{mod}(\beta, 2\Pi)$, $\beta' \in (0, 2\Pi)$. See, e.g., page 1 of Reference [2] which indicates that the angular range must satisfy $\Delta\beta' \in [\Pi+2\gamma m, 2n]$. Data from different rotations are thus combined to form data connected in terms of $\beta'$. The sorted data are therefore consecutive in $\beta'$. See also the right of FIG. 2 in Reference [2]. Note the difference in notation between the notation used in this application and the notation of Reference [2]. Specifically, $\beta' \rightarrow \alpha$ and $2\gamma_m \rightarrow \Phi$.

A weighted fan-beam algorithm based on the Hilbert transform (1) and a weighted parallel-beam ramp-filtering algorithm (2) have also been considered [3]. These algorithms filter data by using the kernel of the Hilbert transform for (1) and by using the kernel of the ramp-filter for (2), weight the filtered data to compensate for redundancy of samples, and backproject the weighted filtered data to obtain images. However, the applicability of these algorithms to reconstruct images from disconnected projection data is subject to major constraints. Specifically, only an algorithm that is not based on ramp-filtering may be used when either a fan-beam or a cone-beam is used. And after applying either a fan-to-parallel beam or a cone-to-fan parallel-beam re-binning technique, only parallel-beam reconstruction may be used.

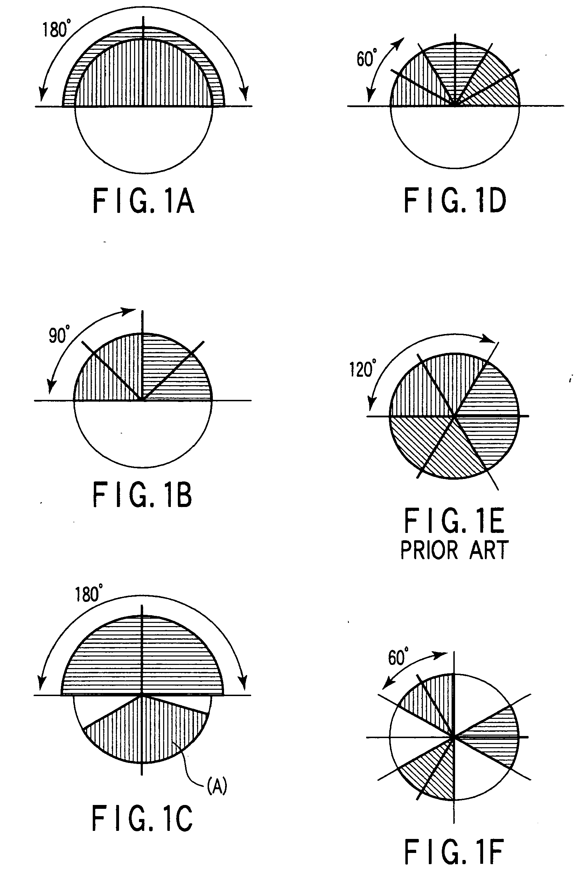

A weighted parallel-beam ramp-filtering algorithm with disconnected projection data for cardiac imaging has been discussed [4,5]. In this case, the data preserve divergence along the z-axis while the xy direction is parallel. (This is also called parallel-fan-beam.) Specifically, Equation 8 in Reference [4] shows cone-beam to parallel-beam re-binning (sorting), FIG. 1 in Reference [4] shows re-binned parallel-beam data, and Equation 20 of Reference [4] and Equation 5 of Reference [5] show that the algorithm uses cyclic angles over 180° (and not 360°). It is natural in the context of these references, which use parallel-beam data, to develop algorithms based on 180° cyclic angular range. However, these algorithms do not extend to fan-beam or cone-beam geometry, wherein projection data is cyclic over 360°. Incorporating the cyclic concept over 360°, as needed with fan-beam or cone-beam geometry, is much more complex than doing so over 180°.

Potentially disconnected projections have been alluded to in the context of using ECG signal data to choose data sets for z-axis interpolation and to calculate weights for such data sets [6]. The weights in Reference [6] are used to perform z-axis interpolation and do not pertain to a compensation for redundancy of samples.

The generation of Taiko data by interpolation has been discussed [7,8]. The generated Taiko data is used to fill in missing data after which standard reconstruction techniques may be used. The Taiko approach of References [7,8] does not, however, discuss a weighting scheme. Moreover, the Taiko approach requires considerable computational processing power, which is a major disadvantage.

An approach with the potential to reconstruct images from disconnected projections by using both a ramp filtering and Hilbert transform has been suggested by the present inventor and his colleagues [9]. However, this approach is in a preliminary stage and has not been proven or fully discussed yet. Moreover, this approach requires two convolutions for each angle $\beta$ which may also require considerable computational power.

The above-mentioned algorithms therefore do not allow one to (1) use data which is not parallel but divergent in the xy plane; (2) use a filtered backprojection algorithm based on ramp-filtering; and (3) use disconnected projection data. The above-mentioned algorithms are also characterized with several other problems. These problems are now further discussed.

The use of fan-beam or cone-beam geometry requires all patches to be connected together in terms of the projection angle so that the patches consecutively form a halfscan range. In this context, two major problems are associated with conventional algorithms: (1) suboptimal temporal resolution and (2) larger image noise.

Conventional algorithms, such as the ones discussed above, do not allow one to use complementary rays which are 180° apart, i.e., $p(\beta,\gamma)=p(\beta+\pi+2\gamma,-\gamma)$, which parallel beam does as $p_p(\theta,t)=p_p(\theta+\pi,-t)$. This constraint limits the temporal resolution and/or increases the image noise.

FIGS. 1A-1F illustrate several configurations of patches. FIG. 1A illustrates 180° patches. FIG. 1B illustrates connected 90° patches. FIG. 1C illustrates one 180° patch flanked on the other side by a 120° patch. FIG. 1D illustrates a case wherein the number of patches is three and each patches are separated by 60° in projection angle. This provides the smallest patch size and the best temporal resolution (1/6 of one rotation, Trot). However, if the patches are separated by 120°, the patch size with the known algorithms is 120° and the temporal resolution is Trot/3, twice as large as the best case. This is illustrated in FIG. 1E. Further, if there is a patch in the opposite side, such as the red patch of FIG. 1C, one has to either (1) discard it which leads to larger image noise or (2) expand the patch to connect it to another patch which may degrade the temporal resolution. FIG. 1F illustrates three disconnected 60° patches, which is the difficult situation the present invention will address.

The algorithms based on either re-binning to parallel beam or re-binning to parallel-fan-beam are problematic because of a risk of artifacts due to motion or other factors. This risk arises because these algorithms filter data obtained at different times.- The re-binning algorithms are also problematic because they require considerable computational processing power.

The algorithms based on the Hilbert transform require additional work on convolution filters despite considerable investments made by the industry to modify and optimize current ramp filters for specific clinical applications such as tissue, abdomen, brain, lung, bone, etc. Methods and algorithms avoiding such additional work directed to a specific application would be very advantageous.

Reference [23] discusses disconnected halfscan. However, this reference constrains the angular intervals of the patches to be equal. No such constraint should be imposed and the angular intervals will be arbitrary in embodiments of the invention.

The second general observation one can make regarding cardiac imaging is that conventional techniques do not allow one to smooth the transition from one heart cycle to another. Moreover, since the lack of smoothness associated with heart cycle transitions is associated with banding artifacts, conventional techniques often yield cardiac imaging artifacts.

FIGS. 2A-2C illustrate examples of artifacts. FIG. 2A illustrates artifacts (indicated by the arrows (a),(b)) in an image obtained using fan-beam computed tomography. FIG. 2B illustrates artifacts (indicated by the arrows (a)) in an image obtained using cone-beam computed tomography. FIG. 2C illustrates a nearly artifact-free image obtained using cone-beam computed tomography in conjunction with the present invention.

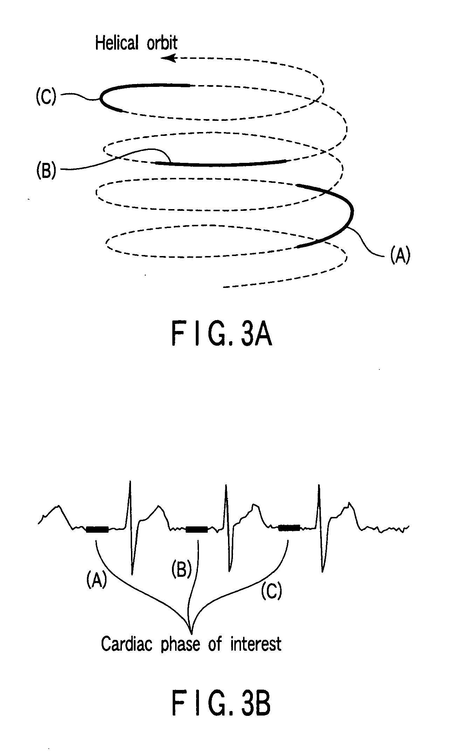

FIGS. 3A and 3B illustrate one of the reasons explaining the presence of artifacts in cardiac imaging. FIG. 3A illustrates a helical orbit wherein projection data is obtained for a cardiac phase of interest (i.e., within a certain cardiac or time window). FIG. 3B illustrates the cardiac phase of interest. The segments (A), (B), (C) represent the same phase for which projection data are obtained to reconstruct images or volume as represented by the disjoint segments (A), (B), (C) in FIG. 3A. Thus, even if the original scanning orbit is continuous (such as a helix), the effective or valid segments (patches) of the orbit are not continuous. Naturally, this situation is unavoidable in helical cardiac imaging given the dynamic nature of the heart.

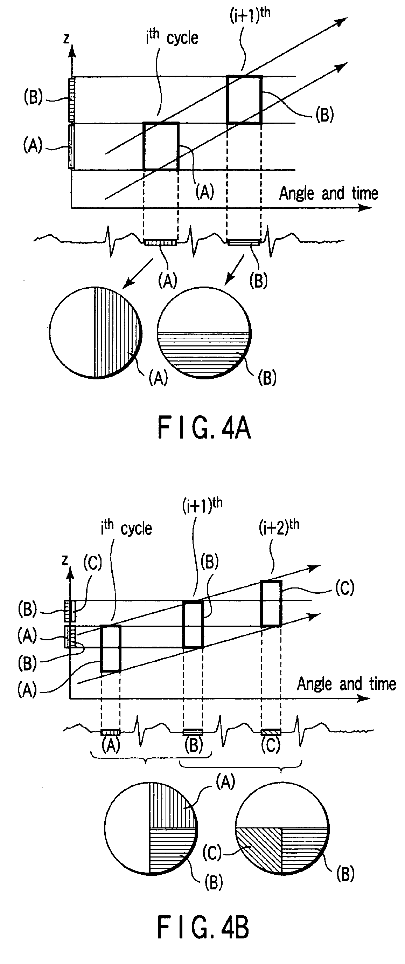

FIGS. 4A and 4B illustrate ideas facilitating an understanding of previously proposed algorithms. FIG. 4A relates to a single-cycle method (SCM). FIG. 4B relates to multi-cycle method (MCM).

SCM uses projection data from one cardiac cycle (or heart beat) to reconstruct one slice (or one slab of the volume) in stacked fan-beam geometry [10,11,12]. The algorithm first interpolates data simultaneously obtained along the z-axis. The algorithm then applies a time window weighting function over a certain period of time (e.g., a halfscan) and reconstructs images with a fan-beam algorithm based on ramp filtering.

SCM can be extended to a cone-beam algorithm, wherein it reduces to a simple, helical Feldkamp halfscan upon adjusting the time center to the gating point (see the time-shifting technique in References [10,11]). Specifically, the gating center is first found, a halfscan weighting is then applied to the projection data centering the gating point, and, lastly, filtered cone-beam backprojection is used.

However, SCM uses one heart cycle (or, more precisely, one cardiac time window or one "patch") to reconstruct one slice or slab of volume. In FIG. 4A, the red heart cycle is used to reconstruct the red slab on the z-axis using about half a rotation; and the blue heart cycle (with a different half a rotation angle) is used to reconstruct the blue slab on the z-axis. References [10,11,12] describe SCM algorithms for fan-beam or stacked fan-beam. Reference [13] uses SCM with a fan-to-parallel re-binning technique.

MCM uses projection data from more than one cardiac cycle to reconstruct one slice by using a narrower time window. In FIG. 4B, the red and blue patches are used to reconstruct the lower slab (displayed in red and blue around the z-axis); and the blue and green cycles are used to reconstruct the upper slab (displayed in blue and green around the z-axis). The MCM algorithm has been discussed in the context of stacked fan-beam geometry [14], cone-beam geometry [2], and fan-to-parallel re-binning based parallel-beam geometry [15,16,17].

Aside from SCM and MCM, an interpolation based method (180MCI) has also been considered. However, the crucial issue of the transition from one heart cycle to another heart cycle has not been addressed, or even discussed, in the context of any of the above mentioned algorithms.

A technique for feathering between patches (cardiac cycles) with overlapping projection angles has been discussed [19,20]. However, whereas this approach aims at reducing the abrupt transition from one cardiac cycle to another cycle, Reference [19] only treats a problem within one detector fan-beam projection data and Reference [20] discusses a problem within one slice caused by abrupt change in projection angles. These developments do not concern the artifacts at issue in the present invention since these artifacts, being caused by a discontinuity along the z-axis from one slice to another slice can not be seen in an axial image.

References [4,5,18] use MCM with a cone-to-parallel fan-beam re-binning technique and discuss an illumination window having a feathering feature at both the beginning and the end of the window. However, this so-called overscan weight has been known for many years (see, e.g., Reference [21]) to compensate for redundant samples. Embodiments of the present invention addressing the artifact problem have nothing to do with the redundancy weighting. Moreover, the trapezoid function used in References [4,5] must have a breaking point at a period of $\pi$ in $\phi$ (the parallel projection angle). Such a restriction constitutes a major disadvantage and can prevent the desired smoothing.

A method to adjust the priority of plural patches including ones by Taiko data to form continuous halfscan range has been discussed [7,8]. However, the results therein abruptly change in z. This method is therefore not pertinent for reducing the artifacts with which the present invention is concerned.

The above-mentioned algorithms therefore do not allow one to smooth the transition from one heart cycle to another and thus to prevent banding artifacts that occur in cardiac imaging. The above-mentioned algorithms have several other problems. These problems are now further discussed.

In particular, there is a problem with horizontal banding artifacts. In the references, the cardiac cycles to use in the reconstruction, and their amount of contribution in reconstruction, are defined totally independently in a slice-by-slice fashion. In fact, they are even defined independently in very discrete, pixel-by-pixel fashion in References [4,5,17]. As illustrated in FIGS. 4A and 4B, there is an abrupt transition between slabs along the z-axis, such as between the red and blue slabs in FIG. 4A, or between the red/blue and the blue/green slabs in FIG. 4B. This abrupt change in the choice and the contribution of each heart cycles in z, is the cause of the horizontal banding artifact shown by the yellow arrows in FIGS. 2A and 2B.

Accordingly, to overcome the problems of the related art, the present invention provides a method, system, and computer program product for reconstructing an image, comprising: (1) obtaining projection data using an X-ray detector and one of a cone-beam X-ray generator and a fan-beam X-ray generator; (2) weighting the projection data to compensate for redundant projection data; (3) filtering the weighted data using a ramp-based filtering function to generate filtered projection data; and (4) reconstructing the image by back-projecting the filtered projection data along a radial path.

Accordingly, to overcome the problems of the related art, the present invention provides a method, system, and computer program product for reconstructing an image, comprising: (1) obtaining projection data using a multi-row X-ray detector and one of a cone-beam X-ray generator and a fan-beam X-ray generator, wherein the projection data are obtained using a helical trajectory; (2) obtaining a physiologic signal having a first physiologic cycle and a second physiologic cycle; (3) determining, based on the obtained projection data and the obtained physiologic signal, first projection data corresponding to the first physiologic cycle and second projection data corresponding to the second physiologic cycle; (4) weighting the first projection data and the second projection data so that a contribution of the first projection data and the second projection data changes gradually along a rotational axis of the helical trajectory; and (5) reconstructing the image from the weighted first projection data and the weighted second projection data.

Accordingly, to overcome the problems of the related art, the present invention provides a method, system, and computer program product for reconstructing an image, comprising: (1) obtaining projection data using an X-ray detector and one of a cone-beam X-ray generator and a fan-beam X-ray generator; (2) weighting the obtained projection data to compensate for redundant projection data; (3) filtering the weighted projection data using a ramp-based filtering function to generate filtered projection data; (4) reconstructing the image by back-projecting the filtered projection data along a radial path.

Accordingly, to overcome the problems of the related art, the present invention provides a method, system, and computer program product for reconstructing an image, comprising: (1) obtaining projection data using a multi-row X-ray detector and one of a cone-beam X-ray generator and a fan-beam X-ray generator, wherein the projection data are obtained using a helical trajectory; (2) obtaining a physiologic signal having a first physiologic cycle and a second physiologic cycle; (3) determining, based on the obtained projection data and the obtained physiologic signal, first projection data corresponding to the first physiologic cycle and second projection data corresponding to the second physiologic cycle; (4) reconstructing first image data and second image data from the first projection data and the second projection data, respectively; (5) weighting the first image data and the second image data so that a contribution of the first image data and the second image data changes gradually along a rotational axis of the helical trajectory; and (6) combining the weighted first image data and the weighted second image data to reconstruct the image.

Accordingly, to overcome the problems of the related art, the present invention provides a method, system, and computer program product for reconstructing an image, comprising: (1) obtaining a plurality of temporally disconnected projection data sets of a scanned object corresponding to a plurality of disjoint projection angle intervals; (2) calculating, based on the obtained projection data sets, and without generating Taiko rays, a plurality of partial images corresponding to the plurality of projection angle intervals, by weighting, for each projection angle interval, projection data corresponding to an interval other than the projection angle interval; and (3) combining the calculated partial images corresponding to each of the plurality of projection angle intervals to generate the image.

Accordingly, to overcome the problems of the related art, the present invention provides a method, system, and computer program product for reconstructing an image, comprising: (1) obtaining a plurality of temporally disconnected projection data sets of a scanned object corresponding to a common movement phase of the scanned object; (2) determining a level of contribution for each obtained projection data set based on a distance from a center of the obtained projection data set; and (3) reconstructing the image based on the obtained plurality of temporally disconnected projection data sets and the corresponding determined levels of contribution.

Accordingly, to overcome the problems of the related art, the present invention provides a system for reconstructing an image, comprising: one of a cone-beam X-ray generator and a fan-beam X-ray generator configured to generate X-rays; an X-ray detector configured to detect X-rays generated by the one of a cone-beam X-ray generator and a fan-beam X-ray generator; and a processor device having an embedded computer program configured to perform the steps of (1) obtaining computed tomography (CT) projection data using the X-ray detector and the and one of a cone-beam X-ray generator and a fan-beam X-ray generator; (2) filtering the obtained CT projection data using a ramp-based filtering function to generate filtered projection data; (3) weighting the filtered projection data to compensate for redundant projection data; and (4) reconstructing the image by back-projecting the weighted projection data along a radial path.

Accordingly, to overcome the problems of the related art, the present invention provides a system for reconstructing an image, comprising: one of a cone-beam X-ray generator and a fan-beam X-ray generator configured to generate X-rays; a multi-row X-ray detector configured to detect X-rays generated by the one of a cone-beam X-ray generator and a fan-beam X-ray generator; a monitoring device configured to obtain a physiologic signal; and a processor device having an embedded computer program configured to perform the steps of (1) obtaining CT projection data using the multi-row X-ray detector and the one of a cone-beam X-ray generator and a fan-beam X-ray generator, wherein the CT projection data is obtained using a helical trajectory; (2) obtaining a physiologic signal having a first physiologic cycle and a second physiologic cycle using the monitoring device; (3) determining, based on the obtained CT projection data and the obtained physiologic signal, first projection data corresponding to the first physiologic cycle and second projection data corresponding to the second physiologic cycle; (4) weighting the first projection data and the second projection data so that a contribution of the first projection data and the second projection data changes gradually along a rotational axis of the helical trajectory; and (5) reconstructing the image from the weighted first projection data and the weighted second projection data.

According to an aspect of the present invention, there is provided a method for using weighted fan-beam or cone-beam ramp-filtering algorithm with disconnected projection data sets. The method is called DIRECT (disconnected projection data redundancy compensation technique). This method can be used to reconstruct images or a volume by using physiologic signals which may include, e.g., signals related to the cardiac motion such as the electrocardiogram (ECG), "Kymogram" signals which represent the shape of heart, signals related to respiratory motion such as mechanical vibrations, chest wall motion, and chest size related electric resistance. The method can be used with.an arbitrary scanning orbit including, but not limited to, helical and circular scan.

According to another aspect of the present invention, there is provided a method which can be used not only in cardiac imaging, but also to compensate for missing data caused, for example, by an error in data transfer. This method can be used with arbitrary scanning orbit, geometry, and sampling scheme. Specifically, the orbit could be helical, circular, circular plus line(s), or have a "saddle" trajectory. The geometry can be fan-beam or cone-beam with flat, curved cylindrical, or spherical detectors. The sampling scheme can be equi-space or equi-angle; and could even be based on non-uniform sampling intervals. The reconstruction technique can be based on fan-beam, approximated stacked-fan-beam, or cone-beam algorithms. The scanner type can be of the 2nd, 3rd, 4th, or 5th generation.

According to another aspect of the present invention, there is provided a method called CBC (cardiac banding artifact correction) which can be used to correct for cardiac banding artifacts that arise when transitions between cardiac cycles are not smooth. This method can be based on either or both of weight and size and can be applied to an arbitrary scanning orbit, but preferably to a non-circular scanning orbit having time dependent z-coverage such as helical scanning. This method is independent of geometry, sampling scheme, and reconstruction techniques. Moreover, this method can be applied not only to ramp filtering based fan-beam or cone-beam algorithms, but also to any other algorithm characterized with cardiac cycle transitions in z. Possible algorithms include, but are not limited to, parallel-beam or parallel-fan-beam algorithms based on ramp filtering, algorithms based on the Hilbert transform, algorithms based on Radon inversion, etc. CBC does not impose constraints on any function used to correct for artifacts. These functions can be arbitrary if it is the best for cardiac cycle to cycle transition. The CBC is also used for other physiological signal correlated image reconstruction algorithms, such as respiratory motion gated helical reconstruction algorithm.

According to another aspect of the present invention, there is provided a method for performing ECG-gated fan-beam and cone-beam helical reconstruction algorithm with DIRECT.

According to another aspect of the present invention, there is provided ECG-gated reconstruction with CBC and DIRECT.

A more complete appreciation of the invention and many of the attendant advantages thereof will be readily obtained as the same becomes better understood by reference to the following detailed description when considered in connection with the accompanying drawings, wherein:

FIGS. 1A-1F illustrate a variety of patch configurations;
FIGS. 2A-2C illustrate the presence of artifacts in some multi-cycle reconstructed images;
FIGS. 3A and 3B illustrate cardiac phases of interest and the associated patches along a scanning orbit;
FIGS. 4A and 4B illustrate the use of single-slice and multi-slice methods;
FIGS. 5A and 5B illustrate a scanning process and reconstruction based on a primary ray;
FIGS. 6A and 6B illustrate a scanning process and Taiko reconstruction;
FIGS. 7A and 7B illustrate a scanning process and reconstruction without generating Taiko rays;
FIGS. 8A and 8B illustrate the extension from a single primary ray to an interval of primary rays;

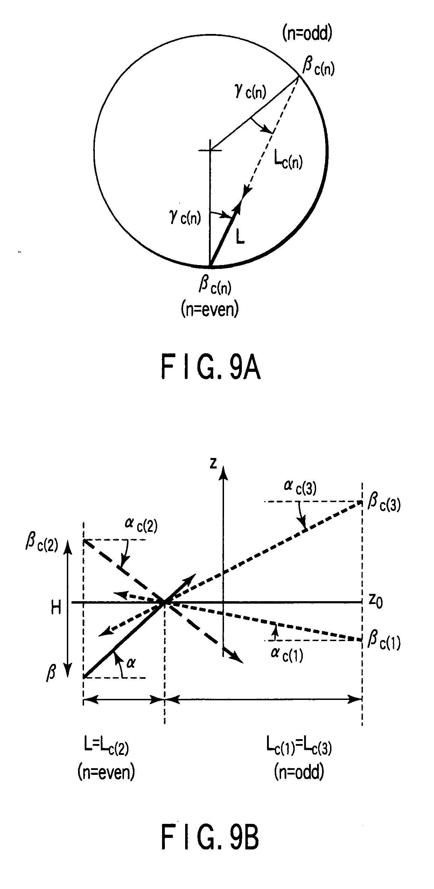

FIGS. 9A and 9B illustrate complementary rays used in normalizing weights;
FIG. 10 illustrates the cone-beam geometry and a cylindrical detector;
FIGS. 11A and 11B illustrate disconnected patches including three optimal patches;
FIGS. 12A-12C illustrate the smoothness between patches that corrects cardiac banding artifacts;
FIG. 13 illustrates a trapezoid function used for correcting cardiac banding artifacts;
FIG. 14 illustrates a method for reconstructing an image;
FIG. 15 illustrates a method for correcting for cardiac banding artifacts; and
FIG. 16 illustrates an improved system for reconstructing an image from temporally disconnected projection data sets.

According to a first embodiment of the present invention, an image $f(x,y)$ can be reconstructed in two dimensions using equations

$$f(x,y) = R/2\pi \int_0^{2\pi} \frac{1}{L(x,y,\beta)^2} \int_{-\gamma_m}^{\gamma_m} \left\{ h(\gamma - \gamma') \left[ w(\beta,\gamma') p(\beta,\gamma') \right] \right\} \, d\gamma' \, d\beta \quad (1)$$

and

$$p(\beta,\gamma) = p(\beta + \pi + 2\gamma, -\gamma), \quad (2)$$

wherein $p(\beta,\gamma)$ denotes a projection datum (i.e., a ray-sum or a line integral) at projection-angle $\beta$ for a rotation angle of the radiation source, and ray-angle $\gamma$ for an angle of each radiation in the fan, $w(\beta,\gamma)$ refers to a weighting function, such as halfscan or fullscan, $h(\bullet)$ is a ramp kernel, $L(x,y,\beta)$ refers to a distance from the focus at $\beta$ to a pixel (x,y), and R is the radius of the orbit. For simplicity, the known weight cos $\gamma$ applied to p for compensating the path length difference among $\gamma$ has not been included and the weights $w(\beta,\gamma)$ are deleted from now on.

A Taiko ray is a datum generated by Equation (2); it is done using bilinear interpolation of the primary rays if the data sample is discrete. However, the objective here is to obtain a partial image from missing data $\beta \in [\beta_1, \beta_2]$ without generating Taiko rays. Note that "partial images" are used here to describe such Taiko data. However, this is done solely to simplify the understanding of the invention and the scope of the invention is in no way limited to the image domain scheme. In fact, as shown in the final results, it is implemented in the projection data domain. Actually, the method in the projection data domain is much simpler to implement.

Consider, as a first step, a partial image corresponding to projection data $\beta = \beta_0$:

$$f(x,y)\big|_{\beta=\beta_0} = \frac{R}{2\pi} \frac{1}{L(x,y,\beta_0)^2} \int_{-\gamma_m}^{\gamma_m} \{h(\gamma-\gamma')p(\beta_0,\gamma')\} \, d\gamma' \, . \qquad (3)$$

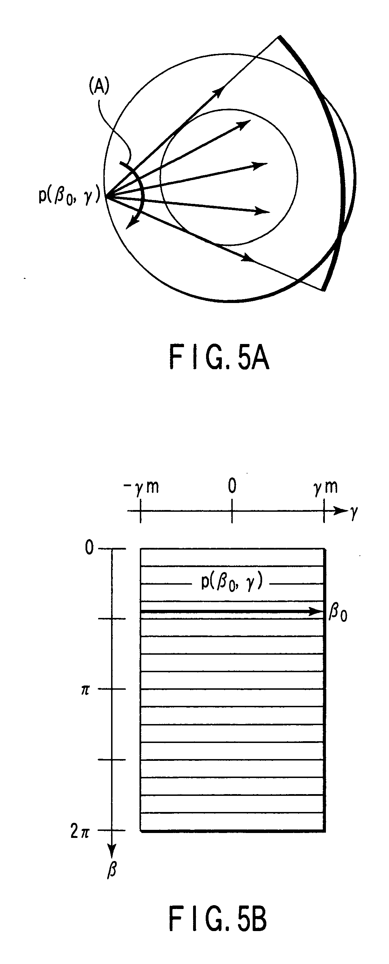

FIG. 5A illustrates an exemplary filtering process for which filtered data is obtained, as shown in FIG. 5B. Equation (3) does the following: (1) filter data in horizontal direction $\beta=\beta_0$, as described by the arrows (A), and (2) apply fan-beam backprojection from one focal spot $\beta=\beta_0$.

Using Equation (2), one obtains

$$f(x,y)\big|_{\beta=\beta_0} = f(x,y)\big|_{\beta=\beta_0}^{GenerateTaiko}$$

$$= \frac{R}{2\pi} \frac{1}{L(x,y,\beta_0)^2} \int_{-\gamma_m}^{\gamma_m} \left[ h(\gamma-\gamma')p^{Taiko}(\beta_0,\gamma') \right] \, d\gamma' \qquad . \quad (4)$$

$$= \frac{R}{2\pi} \frac{1}{L(x,y,\beta_0)^2} \int_{-\gamma_m}^{\gamma_m} \left[ h(\gamma-\gamma')p(\beta_0+\pi+2\gamma',-\gamma') \right] \, d\gamma'$$

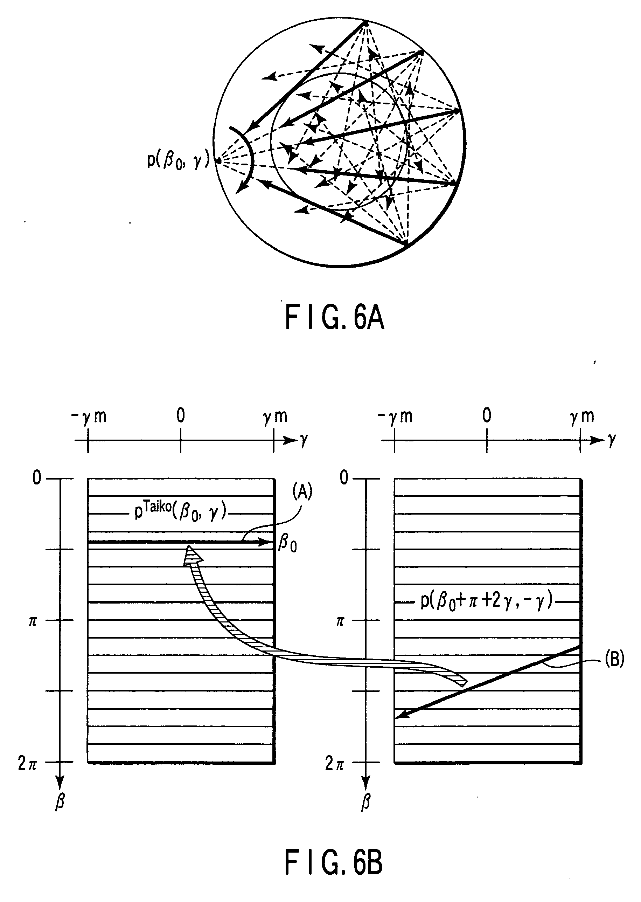

FIG. 6A illustrates an alternative method. FIGS. 6B and 6C illustrate a Taiko strategy wherein one generates $p^{Taiko}(\beta,\gamma)$ from $p(\beta_0+\pi+2\gamma,-\gamma)$ (arrow (B)) using Equation 4 to fill the lost primary $p(\beta_0,\gamma)$ and filter $p^{Taiko}(\beta_0,\gamma)$ in horizontal direction (arrow (A)) . Ramp filtering is done in the horizontal direction. Backprojection is then done at $\beta=\beta_0$. Alternatively, ramp filtering can be done in a diagonal direction $\beta=\beta_0+\pi+2\gamma$. One can then use fan-beam backprojection from $\beta=\beta_0$ only. Therefore, in this case, one replaces primary rays by generated Taiko rays.

However, it turns out that

$$f(x,y)\big|_{\beta=\beta_0} = f(x,y)\big|_{\beta=\beta_0}^{WeightTaiko}$$

$$= \frac{R}{2\pi} \int_{\beta_0+\pi-2\gamma_m}^{\beta_0+\pi+2\gamma_m} \frac{1}{L(x,y,\beta)^2} \int_{-\gamma_m}^{\gamma_m} \left[ h(\gamma-\gamma')n(\beta,\gamma')p(\beta,\gamma') \right] \, d\gamma' \quad d\beta$$

$$(5)$$

where

$$n(\beta,\gamma) = \begin{cases} 1 & if \; \beta = \beta_0 + \pi + 2\gamma \\ 0 & otherwise \end{cases} \, . \qquad (6)$$

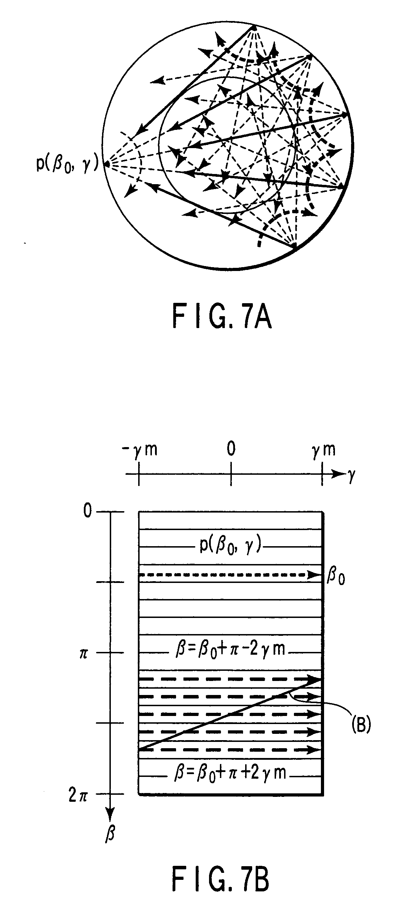

FIG. 7A illustrates another exemplary filtering method. FIG. 7B illustrates another strategy. Equation 5 applies a function $n(\beta,\gamma)$ to primary rays not near $\beta=\beta_0$, but at the opposite side of $\beta=\beta_0$, to only "extract" the necessary Taiko rays (blue lines). Ramp filtering in horizontal ($\gamma$) direction is then used (red dashed arrows); and then fan-beam backprojection for the entire image not at $\beta=\beta_0$, but over some projection angular range $\beta \in [\beta_0 + \pi - 2\gamma_m, \beta_0 + \pi +2\gamma_m]$. Basically, this alternate strategy comprises weighting the primary rays which correspond to the desired Taiko rays. The concept of redundant data samples is used but Taiko rays are not generated. Rather, the corresponding primary rays are simply weighted.

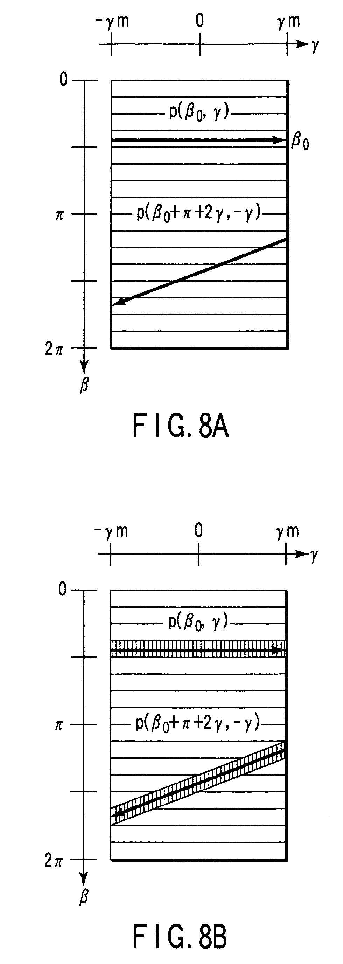

FIGS. 8A and 8B illustrate an extension of this discussion to intervals of rays. Specifically, one can extend this framework from $\beta=\beta_0$, as shown in FIG. 8A, to $\beta\in[\beta_1, \beta_2]$, as shown in FIG. 8B. A partial image can be obtained by primary rays using

$$f(x,y)\big|_{\beta\in[\beta_1,\beta_2]} = R\!\!\Big/\!2\pi \int_{\beta_1}^{\beta_2} \frac{1}{L(x,y,\beta)^2} \int_{-\gamma_m}^{\gamma_m} \big[h(\gamma-\gamma')p(\beta,\gamma')\big]\, d\gamma'\; d\beta, \quad (7)$$

by generating Taiko rays,

$$f(x,y)\big|_{\beta\in[\beta_1,\beta_2]} = f(x,y)\big|_{\beta\in[\beta_1,\beta_2]}^{GenerateTaiko}$$

$$= R\!\!\Big/\!2\pi \int_{\beta_1}^{\beta_2} \frac{1}{L(x,y,\beta)^2} \int_{-\gamma_m}^{\gamma_m} \big[h(\gamma-\gamma')p^{Taiko}(\beta,\gamma')\big]\, d\gamma'\; d\beta ,$$

$$= R\!\!\Big/\!2\pi \int_{\beta_1}^{\beta_2} \frac{1}{L(x,y,\beta)^2} \int_{-\gamma_m}^{\gamma_m} \big[h(\gamma-\gamma')p(\beta+\pi+2\gamma',-\gamma')\big]\, d\gamma'\; d\beta$$

$$(8)$$

or, according to a feature of an embodiment of the invention, using

$$f(x,y)\big|_{\beta\in[\beta_1,\beta_2]} = f(x,y)\big|_{\beta\in[\beta_1,\beta_2]}^{WeightTaiko}$$

$$= R\!\!\Big/\!2\pi \int_{\beta_1+\pi-2\gamma_m}^{\beta_2+\pi+2\gamma_m} \frac{1}{L(x,y,\beta)^2} \int_{-\gamma_m}^{\gamma_m} \big[h(\gamma-\gamma')n(\beta,\gamma')p(\beta,\gamma')\big]\, d\gamma'\; d\beta$$

$$(9)$$

where

$$n(\beta,\gamma) = \begin{cases} 1 & if\ \beta_1+\pi+2\gamma \le \beta \le \beta_2+\pi+2\gamma \\ 0 & otherwise \end{cases} . \quad (10)$$

Complete image reconstruction can then be achieved using (A) consecutive primary rays using

$$f(x,y) = f(x,y)\big|_{\beta\in[0,\beta_1]} + f(x,y)\big|_{\beta\in[\beta_1,\beta_2]} + f(x,y)\big|_{\beta\in[\beta_2,\beta_3]}$$

$$+ f(x,y)\big|_{\beta\in[\beta_3,\beta_4]} + f(x,y)\big|_{\beta\in[\beta_4,\beta_5]} \cdots + f(x,y)\big|_{\beta\in[\beta_i,2\pi]} , \quad (11)$$

$$= f(x,y)\big|_{\beta\in[0,2\pi]}$$

(B) consecutive rays with a mixture of primary or Taiko rays using

$$f(x,y) = f(x,y)\big|_{\beta\in[0,\beta_1]} + f(x,y)\big|_{\beta\in[\beta_1,\beta_2]}^{GenerateTaiko} + f(x,y)\big|_{\beta\in[\beta_2,\beta_3]}$$

$$+ f(x,y)\big|_{\beta\in[\beta_3,\beta_4]}^{GenerateTaiko} + f(x,y)\big|_{\beta\in[\beta_4,\beta_5]} \cdots + f(x,y)\big|_{\beta\in[\beta_i,2\pi]} , \quad (12)$$

and (C) non-consecutive primary rays with weighting using

$$f(x,y) = f(x,y)\Big|_{\beta\in[0,\beta_1]}^{WeightTaiko} + f(x,y)\Big|_{\beta\in[\beta_1,\beta_2]}^{WeightTaiko} + f(x,y)\Big|_{\beta\in[\beta_2,\beta_3]}^{WeightTaiko}$$
$$+ f(x,y)\Big|_{\beta\in[\beta_3,\beta_4]}^{WeightTaiko} + f(x,y)\Big|_{\beta\in[\beta_4,\beta_5]}^{WeightTaiko} \cdots + f(x,y)\Big|_{\beta\in[\beta_i,2\pi]}^{WeightTaiko} \quad . \quad (13)$$
$$= f(x,y)\Big|_{\beta\in[0,2\pi]}^{WeightTaiko}$$

It is desirable to introduce a smoothing weight $wn(\beta,\gamma)$ to avoid abrupt changes in weighted data $n(\beta,\gamma)p(\beta,\gamma)$ since we know that numerically unstable data generate significant artifacts through the convolution process.

Final image reconstruction can advantageously be performed as follows once Conditions (C1-C4) presented below are satisfied:

$$f(x,y) = f(x,y)\Big|_{\beta\in[0,2\pi]}^{WeightTaiko}$$
$$= \frac{R}{2\pi} \int_0^{2\pi} \frac{1}{L(x,y,\beta)^2} \int_{-\gamma_m}^{\gamma_m} \left[ h(\gamma-\gamma') wn(\beta,\gamma') p(\beta,\gamma') \right] \, d\gamma' \, d\beta \quad (14)$$

where

$$wn(\beta,\gamma) = \frac{c(\beta)}{\displaystyle\sum_{j=-\infty}^{\infty} \left[ c(\beta+2\pi j) + c(\beta+\pi(2j+1)+2\gamma) \right]} \quad , \quad (15)$$

$$c(\beta) = \begin{cases} 1 & inside\ of\ an\ arc \\ 0-1 & transition \\ 0 & outside\ of\ an\ arc \end{cases} \quad . \quad (16)$$

Condition C1, also called the "DIRECT condition," is that all the line integrals (ray-sums) through the object must be measured at least once. That is, for any $(\beta,\gamma)$,

$$\sum_{j=-\infty}^{\infty} \left[ c(\beta+2\pi j) + c(\beta+\pi(2j+1)+2\gamma) \right] > 0; \quad j \in integer \quad . \quad (17)$$

However, if only a portion of the object is of interest, such as a region of interest (ROI), the DIRECT condition can be relaxed as follows: (1) all the line integrals (ray-sums) through the ROI must be measured at least once and (2) the projection data are not truncated in the ray-angle ($\gamma$) direction. Equation (17) must then be satisfied within a limited range of $\gamma$.

Condition C2 is that the redundancy of samples is properly compensated for. The sum of weights applied to the data corresponding to the same line (ray-sum) must be one. That is, for any $(\beta,\gamma)$.

$$\sum_{j=-\infty}^{\infty} \left[ wn(\beta+2\pi j) + wn(\beta+\pi(2j+1)+2\gamma) \right] = 1; \quad j \in integer \quad . \quad (18)$$

Note that the weighting function $w(\beta,\gamma)$, which originally compensates for redundant data samples, is replaced by $wn(\beta,\gamma)$.

Condition C3 is that weighting is applied prior to convolution of the ramp kernel.

Condition C4 is that the transition of weights in $\gamma$ is smooth, i.e., the transition does not display abrupt changes, which makes it also smooth in $\beta$.

The searching range for j can be some finite interval $[-J_1, J_2]$ after considering the effective range.

Equations (14), (15), and (17) must be appropriately modified if the projection angular range exceeds $2\pi$. Specifically, Equations (14), (15), and (17) become

$$f(x,y) = \frac{R}{2\pi} \int_{-\beta_m}^{\beta_m} \frac{1}{L(x,y,\beta)^2} \int_{-\gamma_m}^{\gamma_m} \left[ h(\gamma - \gamma') wn(\beta,\gamma') p(\beta,\gamma') \right] d\gamma' \ d\beta \tag{14}'$$

where

$$\sum_{j=-J}^{J} wn\left(\beta_{c(j)}, \gamma_{c(j)}\right) = 1; \quad j \in \text{integer}, \tag{15}'$$

and for any $(\beta,\gamma)$,

$$\sum_{j=-J}^{J} c\left(\beta_{c(j)}\right) > 0; \quad j \in integer. \tag{17}'$$

This approach can be extended to cone-beam geometry. In particular, the "three-dimensional" DIRECT condition becomes Condition C5 stating that any "quasi three-dimensional" line integral through the pixel in the object must be measured at least once. That is, for any $(\beta,\gamma,\alpha)$, .

$$\sum_{j=-J}^{J} c\left(\beta_{c(j)}, \alpha_{c(j)}\right) > 0; \quad j \in integer. \tag{19}$$

The weight is then normalized similarly using

$$\sum_{j=-J}^{J} wn\left(\beta_{c(j)}, \gamma_{c(j)}, \alpha_{c(j)}\right) = 1; \quad j \in \text{integer} \tag{20}$$

where

$$\beta_{c(n)} = \begin{cases} \beta + n\pi + 2\gamma & (n = odd) \\ \beta + 2n\pi & (n = even) \end{cases}; \quad \gamma_{c(n)} = \begin{cases} -\gamma & (n = odd) \\ \gamma & (n = even) \end{cases}, \tag{21}$$

$$\alpha_{c(n)} = \tan^{-1}\left(\left[z_0 - z\left(\beta_{c(n)}\right)\right] \Big/ L_{c(n)}\right) = \tan^{-1}\left[-H\beta_{c(n)}\Big/\left(2\pi L_{c(n)}\right)\right], \tag{22}$$

$$L_{c(n)} = \begin{cases} 2R\cos\gamma - L & (n = odd) \\ L & (n = even) \end{cases}, \quad \text{and} \qquad (23)$$

$$L = \left[ z_0 - z(\beta) \right] / \tan\alpha = -\beta H / (2\pi \tan\alpha). \qquad (24)$$

The DIRECT condition can be relaxed, as discussed before, if only a portion of the object is of interest.

More details about this procedure can be found in Reference [21]. The weights in Reference [6] may be normalized in a manner that appears similar to a processing of weights in embodiments of the present invention. However, the weights in Reference [6] are used to perform z-axis interpolation and do not pertain to a compensation for redundancy of samples. Therefore, the algorithms of Reference [6], despite an apparent similarity, represent algorithms unrelated to the present invention.

The image can then be reconstructed using

$$f(x,y,z) = \frac{R}{2\pi} \int_{-\beta_m}^{\beta_m} \frac{1}{L(x,y,\beta)^2} \int_{-\gamma_m}^{\gamma_m} \left\{ h(\gamma - \gamma') \left[ wn(\beta,\gamma',\alpha) p(\beta,\gamma',\alpha) \right] \right\} d\gamma' \, d\beta. \qquad (25)$$

Alternatively, the weights and the associated "DIRECT condition" can be two-dimensional as shown below. Condition C6 is then that any "quasi 3D" line integral through the pixel must be measured at least once. That is, for any $(\beta,\gamma,\alpha)$,

$$\sum_{j=-J}^{J} c\left( \beta_{c(j)} \right) > 0; \quad j \in integer. \qquad (26)$$

The weights can be normalized using

$$\sum_{j=-J}^{J} wn\left( \beta_{c(j)}, \gamma_{c(j)}, \alpha \right) = 1; \quad j \in integer, \quad \text{and then} \qquad (27)$$

$$f(x,y,z) = \frac{R}{2\pi} \int_{-\beta_m}^{\beta_m} \frac{1}{L(x,y,\beta)^2} \int_{-\gamma_m}^{\gamma_m} \left\{ h(\gamma - \gamma') \left[ wn(\beta,\gamma') p(\beta,\gamma',\alpha) \right] \right\} d\gamma' \, d\beta.$$

$$\qquad (28)$$

Again, note that for simplicity, the $\cos\gamma$ and $\cos\alpha$ weights are not shown in Equations (25) and (28). Note also that J is related to $\beta_m$ which defines the searching range. Further, J can be $\infty$ if c is defined for each z, however this does not yield an optimal processing speed.

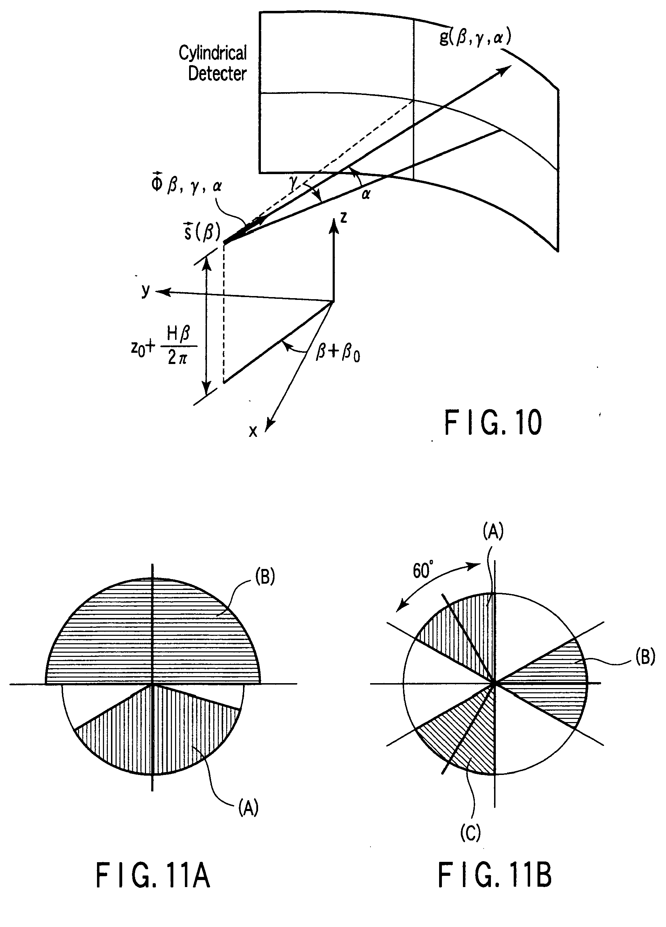

FIG. 11A illustrates patches on opposite sides. DIRECT allows one to use such patches to reduce the image noise and/or to reconstruct images from disconnected patches. Specifically, the patch (B) is used to reconstruct the image and the patch (A) is used to reduce the noise. FIG. 11B illustrates disconnected patches providing the best temporal resolution. Unlike the prior art, DIRECT can process such a configuration of patches and reconstruct the image using all three disconnected patches. Only the central ray corresponding to $\gamma = 0$ is depicted for simplicity. It is perfectly valid for the field of view to be extremely small. The colored areas designate a patch.

According to a second embodiment of the present invention, an ECG-gated reconstruction algorithm based on DIRECT is provided.

The algorithm is discussed using helical scanning as an example. However, this is in no way limiting and the choice of the scanning mode or orbit is arbitrary. For example, it could also be a continuous circular scan ($\beta > 2\pi$).

Further, the physiologic information is not limited to an ECG signal and could be any signal, such as, e.g.,

signals related to cardiac or respiratory motion.

A. cone-beam reconstruction algorithm is also used herein. FIG. 10 illustrates the cone-beam geometry. However, the choice of reconstruction algorithm is also arbitrary and not limited to cone-beam geometry. The algorithm could also be a stacked fan-beam algorithm, for example. One simply has to select an appropriate DIRECT condition for the chosen reconstruction algorithm. Gating points and available patches for the slice of interest are now defined.

A cone-beam projection measured along a helical orbit is given bv

$$g(\beta,\gamma,\alpha) = \int_0^\infty f\big(\bar{s}(\beta) + l\bar{\phi}_{\beta,\gamma,\alpha}\big) dl \quad \text{and} \qquad (29)$$

$$\bar{s}(\beta) = \big(R\cos(\beta+\beta_0),\, R\sin(\beta+\beta_0),\, z_0 + H\beta/(2\pi)\big)^T \qquad (30)$$

where $f(r)$ is the object to reconstruct, R is the radius of the helical orbit, H is the helical pitch (table feed per rotation), $(\beta,\gamma,\alpha)$ denote projection-angle, ray-angle, and cone-angle, respectively (see FIG. 10), and $\phi_{\beta,\gamma,\alpha}$ denotes the unit vector, which is directed from the x-ray focus $s(\beta)$ toward the point $(\gamma,\alpha)$ on the cylindrical detector surface at $\beta$ defined by

$$\bar{\phi}_{\beta,\gamma,\alpha} = \big(-\cos(\beta+\beta_0+\gamma)\cos\alpha,\, -\sin(\beta+\beta_0+\gamma)\cos\alpha,\, \sin\alpha\big)^T. \qquad (31)$$

At $\beta=0$, the focus is in the plane of interest $z=z_0$ at projection angle $\beta_0$. The relative time variable t is zero at the slice of interest $z_0$ and the time-center of the slice at z is defined by

$$t = (\beta-\beta_0)\cdot T_{rot}/(2\pi). \qquad (32)$$

The angular and temporal ranges of projection data for the slice of interest, are bounded by

$$2\beta_m = 2\pi s D/H\,; \qquad (33)$$

$$t_m = \beta_m \cdot T_{rot}/(2\pi)\,; \qquad (34)$$

$$\alpha_m = \tan^{-1}\big[sD/(2R)\big]\,; \quad \text{and} \qquad (35)$$

$$\gamma_m = \sin^{-1}\big(r_0/R\big), \qquad (36)$$

where $2\beta_m$ is the data range used for reconstructing the image at $z=z_0$, $\gamma_m$ is the maximum fan-angle, $\alpha_m$ is the maximum cone-angle to the edge of the detector, $r_0$ is the radius of the cylindrical support of the object, D is the total detector height at the iso-center, and s is the "detector expansion factor," which defines the height of a virtual detector expanded in z with unmeasured data in the expanded part.

If the gating point of each heart cycle lies within the $2\beta_m$ range, then it is used for reconstructing the image at $z=z_0$. One defines a time window, or "patch," centering each gating point with a certain width in time t (thus, in projection angle $\beta$). Let Npatch denote the number of patches within $2\beta_m$ and let ip be the patch index from 0 to Npatch - 1. One then has

$$c(ip,\beta) = \begin{cases} 1 & \textit{if inside of patch ip} \\ 0 & \textit{otherwise} \end{cases} . \qquad (37)$$

To satisfy the "DIRECT" condition, one must ensure that all rays are measured at least once within at least one patch: That is, for any $(\beta,\gamma,\alpha)$,

$$\sum_{j=-\infty}^{\infty} \sum_{ip=0}^{Npatch} c\left(ip,\beta_{c(j)}\right) > 0; \quad j \in integer . \qquad (38)$$

The size (angular range) of each patch has to be adjusted to satisfy Equation (38). Note that the DIRECT condition is independent of the manner of adjustment. One could use another embodiment of the present invention for this purpose. This will be discussed later. This adjustment part is rarely discussed in the literature. Reference [20] adjusts the patch size so that all patches contributing to the slice of interest have the same size.

A feathering technique is then applied to the inside of the edges of each patch. For example, in two dimensions, one may use:

$$c(ip,\beta) = \begin{cases} 0 & \textit{if } \beta < \beta s(ip) \\ rising\left[(\beta - \beta s(ip))/\beta_f\right] & \textit{if } \beta s(ip) \le \beta \le \beta s(ip) + \beta_f \\ 1 & \textit{if } \beta s(ip) + \beta_f < \beta < \beta e(ip) - \beta_f \; ; \quad (39) \\ rising\left[(\beta e(ip) - \beta)/\beta_f\right] & \textit{if } \beta e(ip) - \beta_f \le \beta \le \beta e(ip) \\ 0 & \textit{if } \beta e(ip) < \beta \end{cases}$$

$$rising(x) = \begin{cases} 0 & \textit{if } x \le 0 \\ 3 \times x^2 - 2 \times x^3 & \textit{if } 0 < x < 1, \\ 1 & \textit{otherwise} \end{cases} \qquad (40)$$

where $\beta s(ip)$ and $\beta e(ip)$ are the angles corresponding to the beginning and the end of patch ip, respectively, and $\beta_f$ is the feathering angular range.

Alternatively, in three dimensions, one may use:

$$c(ip,\beta,\alpha) = \begin{cases} 0 & \textit{if } \beta < \beta s(ip) \\ triangle(\alpha/\alpha_m) \times rising\left[(\beta - \beta s(ip))/\beta_f\right] & \textit{if } \beta s(ip) \le \beta \le \beta s(ip) + \beta_f \\ triangle(\alpha/\alpha_m) & \textit{if } \beta s(ip) + \beta_f < \beta < \beta e(ip) - \beta_f \; ; \\ triangle(\alpha/\alpha_m) \times rising\left[(\beta e(ip) - \beta)/\beta_f\right] & \textit{if } \beta e(ip) - \beta_f \le \beta \le \beta e(ip) \\ 0 & \textit{if } \beta e(ip) < \beta \end{cases}$$

$$(41)$$

$$triangle(x) = \begin{cases} 1 - |x| & if\ |x| \le 1 \\ 0 & otherwise \end{cases} . \tag{42}$$

In Equation (41), the denominator of the argument of the triangle function does not have to be $\alpha_m$. Moreover, the triangle function used therein is only an example. For instance, the function itself could also be Gaussian, trapezoid, etc.

The normalized weight are then calculated using

$$wn(\beta, \gamma, \alpha) = \frac{\sum\limits_{ip=0}^{Npatch-1} c(ip, \beta, \alpha)}{\sum\limits_{ip=0}^{Npatch-1} \sum\limits_{j=-\infty}^{\infty} \left[ c\left(ip, \beta_{c(j)}, \alpha_{c(j)}\right) \right]} . \tag{43}$$

Reconstruction can then be accomplished using

$$\tilde{g}(\beta, \gamma, \alpha) = \cos\zeta(\gamma, \alpha)\, wn(\beta, \gamma, \alpha)\, g(\beta, \gamma, \alpha) , \tag{44}$$

$$\cos\zeta(\gamma, \alpha) = \vec{\varphi}_{\beta,\gamma,\alpha} \cdot \vec{\varphi}_{\beta,0,0} , \tag{45}$$

and

$$f(\vec{r})\Big|_{z=z_0} = \frac{1}{2}\pi \int_{-\beta_m}^{+\beta_m} \frac{R}{\left[(\vec{r} - \vec{s}(\beta)) \cdot \vec{\varphi}_{\beta,0,0}\right]^2} \int_{-\infty}^{\infty} \left[ h(\gamma - \gamma')\, \tilde{g}(\beta, \gamma', \alpha) \right]\, d\gamma'\, d\beta . \tag{46}$$

According to a third embodiment of the present invention, a cardiac banding artifact correction technique (CBC) is provided to reduce the effect of transition from one heart cycle to another heart cycle.

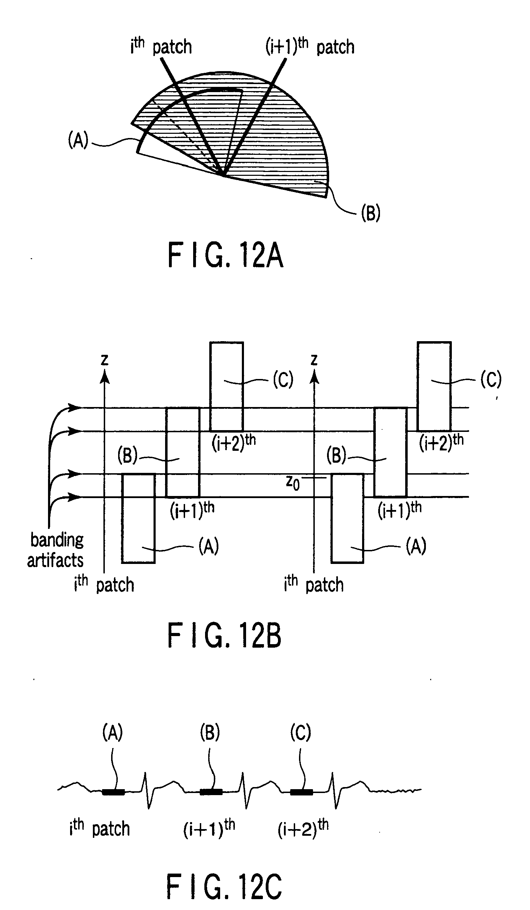

FIGS. 12A-12C illustrate an example of the CBC concept. FIG. 12C shows the ECG signal wherein colored bold lines indicate patches, i.e., cardiac time window, gate window, or phase of interest in each heart cycle (or, heartbeat). FIG. 12B shows the corresponding z coverage of each patch with the same colored box. The darkness (grayscale) inside of each box represents the contribution of each patch to the slice at z. A darker shade of gray indicates a larger contribution to the slice at z. CBC is not applied in the left of FIG. 12B and the contributions of each patch display abrupt changes which yield banding artifacts. For example, the contribution of the blue (i+1)$^{th}$ path changes from 0 to 0.5 to 1 to 0.5 to 0 in discrete steps. The situation can even be worse without using CBC as sometimes changes from 0 to 1 to 0 can occur if one patch is chosen to be used in an overlapped region. It is very susceptible to even a subtle change of heart rate; it is the main cause of the banding artifact. However, the right of FIG. 12B shows what happens upon using CBC. Smooth transitions in the contribution of each heart cycle or patch then arise. That is, the contribution (darkness) of each patch smoothly changes from 0 to 1 to 0 in continuous fashion. This can be achieved by changing either or both of the size and the weight of each patch as a function of a distance from the "center" along the z-axis. The center is defined by the z-coordinate at the center of the z-coverage of each patch. The distance is measured by the physical distance from the center to the z coordinate of the slice of interest.

Alternatively, the center and distance can be defined in terms of a projection angle. The center is then the angle P for which the focal spot is at the slice of interest and the distance to each patch is the angular range from the center to each projection angle $\beta$ or the angle $\beta$ corresponding to the gating point (or the center of each patch).

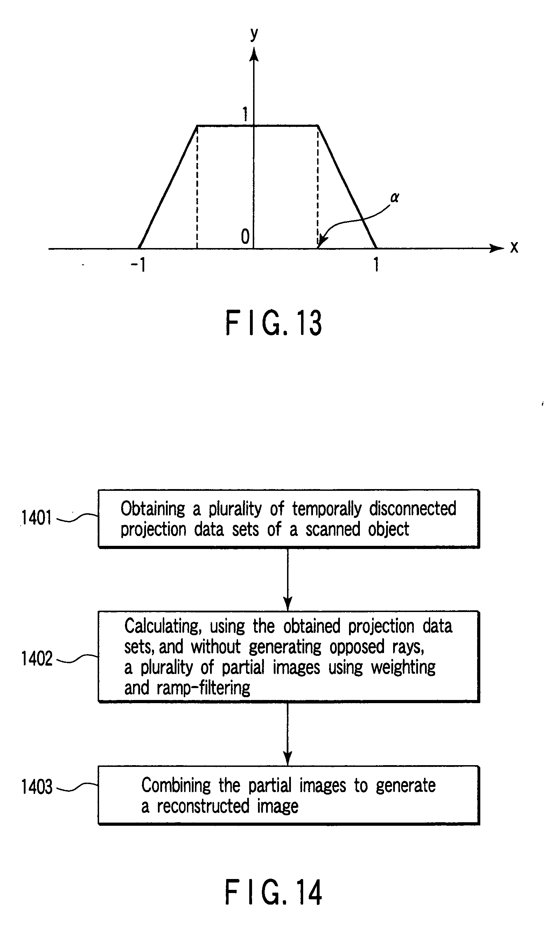

FIG. 13 illustrates a trapezoid function which one might use. For example, one can define this function as:

$$trapezoid(x,a) = \begin{cases} 1 & (|x| \le a) \\ 0 & (|x| \ge 1) \\ 1 - \dfrac{x-a}{1-a} & otherwise \end{cases} \qquad (47)$$

The "ratios" of each patch in size and weight can then be independently defined by

$$wWeight(ip) = \begin{cases} trapezoid\left(\left|z_p(ip) - z_0\right| / zw, aw\right) & z \text{ coordinate based method} \\ trapezoid\left(\left|\beta_p(ip) - \beta_0\right| / \beta w, aw\right) & \beta \text{ based method} \end{cases}$$

and (48)

$$wSize(ip) = \begin{cases} trapezoid\left(\left|z_p(ip) - z_0\right| / zs, as\right) & z \text{ coordinate based method} \\ trapezoid\left(\left|\beta_p(ip) - \beta_0\right| / \beta s, as\right) & \beta \text{ based method} \end{cases}$$

(49)

where $zw$ and $\beta w$ are the widths of the bottom of the trapezoid function along z-axis or β-axis, respectively, $aw$ and $as$ are parameters that define the breaking point (or the shoulder of the flat region in the trapezoid), respectively, and $z_p$ (ip) and $\beta_p$(ip) are the z-coordinate and projection angle for which the focal spot is at the center of patch ip.

Then, let the sizes of a plurality of patches contributing to the reconstruction of the slice at $z=z_0$, be wSize(ip) : wSize(ip+1) : wSize(ip+2) : ... This can be achieved by starting a zero-width patch at each gating point, adjusting the patch expansion increment to the ratio above, and repeating the iterative patch expansion until the DIRECT condition is satisfied.

FIG. 12A illustrates the result, which is that the size (i.e., the contribution) of each patch smoothly changes along z. Specifically, at $z_0$, where the patch

(A) is close to the edge of its coverage and the patch
(B) is close to its center, the patch (A) has a much smaller size than the patch (B). The red patch has smaller weight than the patch (B) in the overlapped angles.

For a cone-beam geometry, one replace Equation (43) with

$$wn(\beta, \gamma, \alpha) = \frac{\displaystyle\sum_{ip=0}^{Npatch-1} wWeight(ip) \cdot c(ip, \beta, \alpha)}{\displaystyle\sum_{ip=0}^{Npatch-1} \sum_{j=-\infty}^{\infty} \left[ wWeight(ip) \cdot c\left(ip, \beta_{c(j)}, \alpha_{c(j)}\right) \right]} . \qquad (50)$$

For a fan-beam geometry, or two dimensional weighting, one uses

$$wn(\beta,\gamma) = \frac{\sum_{ip=0}^{Npatch-1} wWeight(ip)\cdot c(ip,\beta)}{\sum_{ip=0}^{Npatch-1}\sum_{j=-\infty}^{\infty}\left[wWeight(ip)\cdot c\left(ip,\beta_{c(j)}\right)\right]} \cdot \tag{51}$$

Note that the above embodiment of CBC defines the size and weight <u>for each patch</u> rather than for each source point $\beta$ or for each ray $(\beta,\gamma)$.

CBC can also be modified by using

$$wWeight(ip,\beta) = \begin{cases} trapezoid\left(\left|z(\beta)-z_0\right|/zw, aw\right) & z \text{ coordinate based method} \\ trapezoid\left(\left|\beta-\beta_0\right|/\beta w, aw\right) & \beta \text{ based method} \end{cases} \qquad \text{and} \tag{52}$$

$$wn(\beta,\gamma,\alpha) = \frac{\sum_{ip=0}^{Npatch-1} wWeight\left(\beta_{c(j)}\right)\cdot c(ip,\beta,\alpha)}{\sum_{ip=0}^{Npatch-1}\sum_{j=-\infty}^{\infty}\left[wWeight\left(\beta_{c(j)}\right)\cdot c\left(ip,\beta_{c(j)},\alpha_{c(j)}\right)\right]} \cdot \tag{53}$$

Note that the functions used in CBC have nothing to do with the redundancy weighting. Also, the trapezoid function in References [4,5] must have a breaking point at the period of $\pi$ in $\phi$ (the parallel projection angle) whereas the trapezoid used herein, or any other function in CBC, has no such restriction. In fact, the shape of the function can be arbitrary. The best such functions are those that best suppress the effect of the transition from one cardiac cycle to another cycle, but no functions are excluded.

Note also that no specific order in weighting and reconstructing is required. Weighting could be applied first and be followed by reconstruction. Alternatively, images corresponding to the different patches could be reconstructed first and then combined by weighting.

Note also that although the third embodiment describes CBC with DIRECT algorithm, CBC can also be used with other algorithms. For example, CBC could be used with algorithms using connected patches with direct fan-beam or cone-beam algorithm or algorithms based on fan-to-parallel beam re-binning or cone-to-parallel fan-beam re-binning.

According to a fourth embodiment of the present invention, CBC is used in continuous circular dynamic scanning.

The scanning orbit can be described by

$$\vec{s}(\beta) = \left(R\cos(\beta+\beta_0), R\sin(\beta+\beta_0), z_0\right)^T \tag{54}$$

One may then define $\beta_m$ depending on the speed of the time variation of interest. For example, for depicting contrast enhanced blood flow from artery to vein in head with pulsation, a time period corresponding to $2\beta_m$ could be approximately 2 seconds.

The distance and the center for CBC in this case can be along a time axis. Specifically, the center is defined by the time center of interest in cine mode $t_c$, and the distance is measured by the distance along the time axis from the center to the projection angle P corresponding to the center of patch or each projection angle.

For example, one can calculate the angular center $\beta_c$ by using $t_c$:

$$\tilde{t} = \left(\tilde{\beta}-\beta_0\right)\cdot T_{rot}\big/(2\pi)$$
$$\tilde{t}_c = \left(\tilde{\beta}_c-\beta_0\right)\cdot T_{rot}\big/(2\pi) \qquad\qquad , \tag{55}$$

Then, replacing β by β-β$_c$ allows one to reduce the discontinuity along time-axis.

FIG. 14 illustrates a method for reconstructing an image according to embodiments of the present invention. In step 1401, computed tomography projection data are obtained using an X-ray detector and one of a cone-beam or fan-beam X-ray generator. In step 1402, the obtained projection data are weighted to compensate for redundant projection data. In step 1403, the weighted projection data are filtered using ramp-based filtering. In step 1404, the image is reconstructed by back-projecting the weighted projection data along a ray path.

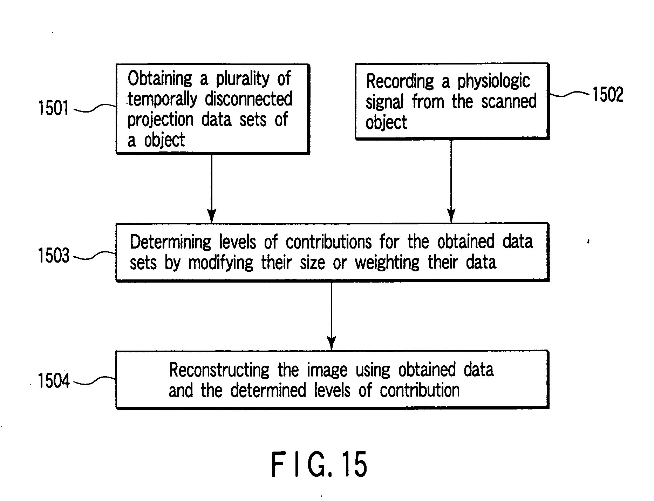

FIG. 15 illustrates a method for correcting cardiac banding artifacts according to embodiments of the present invention. In step 1501, computed tomography projection data are obtained using a multi-row X-ray detector and generator. In step 1502, which occurs in parallel with step 1501, a physiologic signal having first and second physiologic cycles is obtained. In step 1503, first and second projection data corresponding to the first and second physiologic cycles are determined. In step 1503, the first and second projection data are weighted so that their contribution changes gradually along a rotational axis of the helical trajectory. In step 1504, the image is reconstructed using the weighted first and second projection data. Note that the method illustrated in FIG. 2 is based, for example, on a pair of projection. However, this is not limiting in any way and the method naturally expands to a plurality of projection data and physiologic cycles.

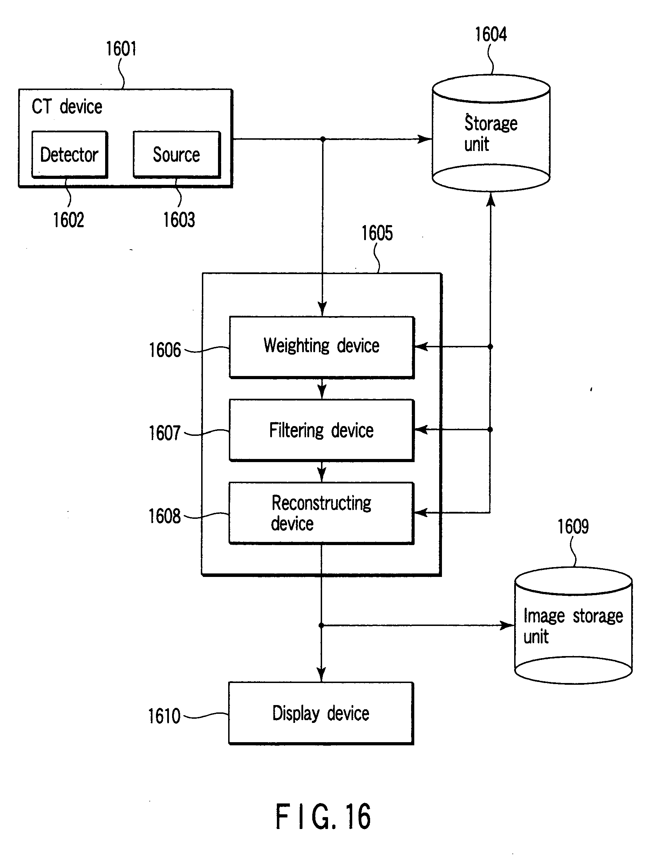

FIG. 16 illustrates a system for carrying out embodiments of the present invention. A computed tomography (CT) device 1601 comprising a ray detector 1602 and a ray source 1603 are used to acquire temporally disconnected CT data. The data can be stored using a storage unit 1604. The data can be processed by a computing unit 1605 which includes a weighting device 1606, a filtering device 1607, and a reconstructing device 1608, to reconstruct a scanned object from the acquired disconnected projection data sets. The weighting device may also be configured to correct for cardiac banding artifacts. The system further comprises an image storing unit 1609 to store images obtained using the data and computing unit and a display device 1610 to display those same images.

All embodiments of the present invention conveniently may be implemented using a conventional general purpose computer or micro-processor programmed according to the teachings of the present invention, as will be apparent to those skilled in the computer art. Appropriate software may readily be prepared by programmers of ordinary skill based on the teachings of the present disclosure, as will be apparent to those skilled in the software art. In particular, the computer housing may house a motherboard that contains a CPU, memory (e.g., DRAM, ROM, EPROM, EEPROM, SRAM, SDRAM, and Flash RAM), and other optional special purpose logic devices (e.g., ASICS) or configurable logic devices (e.g., GAL and reprogrammable FPGA). The computer also includes plural input devices, (e.g., keyboard and mouse), and a display card for controlling a monitor. Additionally, the computer may include a floppy disk drive; other removable media devices (e.g. compact disc, tape, and removable magneto-optical media); and a hard disk or other fixed high density media drives, connected using an appropriate device bus (e.g., a SCSI bus, an Enhanced IDE bus, or an Ultra DMA bus). The computer may also include a compact disc reader, a compact disc reader/writer unit, or a compact disc jukebox, which may be connected to the same device bus or to another device bus.

Examples of computer readable media associated with the present invention include compact discs, hard disks, floppy disks, tape, magneto-optical disks, PROMs (e.g., EPROM, EEPROM, Flash EPROM), DRAM, SRAM, SDRAM, etc. Stored on any one or on a combination of these computer readable media, the present invention includes software for controlling both the hardware of the computer and for enabling the computer to interact with a human user. Such software may include, but is not limited to, device drivers, operating systems and user applications, such as development tools. Computer program products of the present invention include any computer readable medium which stores computer program instructions (e.g., computer code devices) which when executed by a computer causes the computer to perform the method of the present invention. The computer code devices of the present invention may be any interpretable or executable code mechanism, including but not limited to, scripts, interpreters, dynamic link libraries, Java classes, and complete executable programs. Moreover, parts of the processing of the present invention may be distributed (e.g., between (1) multiple CPUs or (2) at least one CPU and at least one configurable logic device) for better performance, reliability, and/or cost. For example, an outline or image may be selected on a first computer and sent to a second computer for remote diagnosis.

Numerous modifications and variations of the present invention are possible in light of the above teachings. It is therefore to be understood that within the scope of the appended claims, the invention may be practiced otherwise than as specifically described herein.

It is explicitly stated that all features disclosed in the description and/or the claims are intended to be disclosed separately and independently from each other for the purpose of original disclosure as well as for the purpose of restricting the claimed invention independent of the composition of the features in the embodiments and/or the claims. It is explicitly stated that all value ranges or indications of groups of entities disclose every possible intermediate value or intermediate entity for the purpose of original disclosure as well as for the purpose of restricting the claimed invention, in particular as limits of value ranges.

**EP 1 699 014 A2**

**Claims**

1. A method for reconstructing an image, **characterized by** comprising:

   obtaining (1401) projection data using an X-ray detector and one of a cone-beam X-ray generator and a fan-beam X-ray generator;
   weighting (1402) the obtained projection data to compensate for redundant projection data;
   filtering (1402) the weighted projection data using a ramp-based filtering function to generate filtered projection data; and
   reconstructing (1403) the image by back-projecting the filtered projection data along a radial path.

2. A method for reconstructing an image, **characterized by** comprising:

   obtaining (1401) projection data using an X-ray detector and one of a cone-beam X-ray generator and a fan-beam X-ray generator;
   filtering (1402) the obtained projection data using a ramp-based filtering function to generate filtered projection data;
   weighting (1402) the filtered projection data to compensate for redundant projection data; and
   reconstructing (1403) the image by back-projecting the weighted projection data along a radial path.

3. The method of claim 1, **characterized in that** the obtaining step comprises:

   obtaining the projection data using the cone-beam X-ray generator.

4. The method of claim 1, **characterized in that** the obtaining step comprises:

   obtaining the projection data using an X-ray generator and a helical scan trajectory.

5. The method of claim 1, **characterized in that** the obtaining step comprises:

   obtaining angularly disconnected projection data.

6. The method of claim 1, **characterized in that** the filtering step comprises:

   filtering the obtained projection data using horizontal ramp-filtering.

7. The method of claim 1, **characterized in that** the filtering step comprises:

   filtering the obtained projection data using diagonal ramp-filtering.

8. The method of claim 1, **characterized in that** the weighting step comprises:

   weighting projection data corresponding to Taiko rays without generating the Taiko rays.

9. A method for reconstructing an image, **characterized by** comprising:

   obtaining (1501) projection data using a multi-row X-ray detector and one of a cone-beam X-ray generator and a fan-beam X-ray generator, wherein the projection data is obtained using a helical trajectory;
   obtaining (1502) a physiologic signal having a first physiologic cycle and a second physiologic cycle;
   determining (1503), based on the obtained projection data and the obtained physiologic signal, first projection data corresponding to the first physiologic cycle and second projection data corresponding to the second physiologic cycle;
   weighting (1503) the first projection data and the second projection data so that a contribution of the first projection data and the second projection data changes gradually along a rotational axis of the helical trajectory; and
   reconstructing (1504) the image from the weighted first projection data and the weighted second projection data.

10. A method for reconstructing an image, **characterized by** comprising:

obtaining (1501) projection data using a multi-row X-ray detector and one of a cone-beam X-ray generator and a fan-beam X-ray generator, wherein the projection data is obtained using a helical trajectory;

obtaining (1502) a physiologic signal having a first physiologic cycle and a second physiologic cycle;

determining (1503), based on the obtained projection data and the obtained physiologic signal, first projection data corresponding to the first physiologic cycle and second projection data corresponding to the second physiologic cycle;

reconstructing (1504) first image data and second image data from the first projection data and the second projection data, respectively;

weighting (1503) the first image data and the second image data so that a contribution of the first image data and the second image data changes gradually along a rotational axis of the helical trajectory; and

combining (1503) the weighted first image data and the weighted second image data to reconstruct the image.

11. The method of claim 9, **characterized in that** the step of obtaining the projection data comprises:

obtaining the projection data using the cone-beam X-ray generator.

12. The method of claim 9, **characterized in that** the step of obtaining a physiologic signal comprises:

obtaining a heart beat signal.

13. The method of claim 9, **characterized in that** the step of obtaining a physiologic signal comprises:

obtaining a respiratory signal.

14. The method of claim 9, **characterized in that** the weighting step comprises:

adjusting a size of the first projection data and a size of the second projection data based on the obtained physiologic signal.

15. The method of claim 9, **characterized in that** the weighting step comprises:

assigning a first weight to each projection datum in the first projection data; and
assigning a second weight to each projection datum in the second projection data.

16. The method of claim 9, **characterized in that** the weighting step comprises:

assigning a different weight to each projection datum in the first projection data; and
assigning a different weight to each projection datum in the second projection data.

17. A system for reconstructing an image, **characterized by** comprising:

an unit (1601) configured to obtain projection data using an X-ray detector and one of a cone-beam X-ray generator and a fan-beam X-ray generator;
an unit (1607) configured to filter the obtained projection data using a ramp-based filtering function to generate filtered projection data;
an unit (1606) configured to weight the filtered projection data to compensate for redundant projection data; and
an unit (1608) configured to reconstruct the image by back-projecting the weighted projection data along a radial path.

18. A system for reconstructing an image, **characterized by** comprising:

an unit (1601) configured to obtain projection data using an X-ray detector and one of a cone-beam X-ray generator and a fan-beam X-ray generator;
an unit (1606) configured to weight the obtained projection data to compensate for redundant projection data;
an unit (1607) configured to filter the weighted projection data using a ramp-based filtering function to generate filtered projection data; and
an unit (1608) configured to reconstruct the image by back-projecting the filtered projection data along a radial path.

**19.** A system for reconstructing an image, **characterized by** comprising:

an unit (1601) configured to obtain projection data using a multi-row X-ray detector and one of a cone-beam X-ray generator and a fan-beam X-ray generator, wherein the projection data is obtained using a helical trajectory;

an unit (1601) configured to obtain a physiologic signal having a first physiologic cycle and a second physiologic cycle;

an unit (1605) configured to determine, based on the obtained projection data and the obtained physiologic signal, first projection data corresponding to the first physiologic cycle and second projection data corresponding to the second physiologic cycle;

an unit (1606) configured to weight the first projection data and the second projection data so that a contribution of the first projection data and the second projection data changes gradually along a rotational axis of the helical trajectory; and

an unit (1608) configured to reconstruct the image from the weighted first projection data and the weighted second projection data.

**20.** A system for reconstructing an image, **characterized by** comprising:

an unit (1601) configured to obtain projection data using a multi-row X-ray detector and one of a cone-beam X-ray generator and a fan-beam X-ray generator, wherein the projection data is obtained using a helical trajectory;

an unit (1601) configured to obtain a physiologic signal having a first physiologic cycle and a second physiologic cycle;

an unit (1605) configured to determine, based on the obtained projection data and the obtained physiologic signal, first projection data corresponding to the first physiologic cycle and second projection data corresponding to the second physiologic cycle;

an unit (1608) configured to reconstruct first image data and second image data from the first projection data and the second projection data, respectively;

an unit (1606) configured to weight the first image data and the second image data so that a contribution of the first image data and the second image data changes gradually along a rotational axis of the helical trajectory; and

an unit (1605) configured to combine the weighted first image data and the weighted second image data to reconstruct the image.

FIG. 1A

FIG. 1D

FIG. 1B

FIG. 1E
PRIOR ART

FIG. 1C

FIG. 1F

(a)

(b)
(h)
(b)

(a)
(a)

FIG. 2A

FIG. 2B

FIG. 2C

Helical orbit

(C)

(B)

(A)

F I G. 3A

(A)

(B)

(C)

Cardiac phase of interest

F I G. 3B

FIG. 4A

FIG. 4B

FIG.5A

FIG.5B

F I G. 6A

F I G. 6B

F I G. 7A

F I G. 7B

F I G. 8A

F I G. 8B

FIG. 9A

FIG. 9B

FIG. 10

FIG. 11A

FIG. 11B

**F I G. 12A**

**F I G. 12B**

**F I G. 12C**

**F I G. 13**

| 1401 | Obtaining a plurality of temporally disconnected projection data sets of a scanned object |
| 1402 | Calculating, using the obtained projection data sets, and without generating opposed rays, a plurality of partial images using weighting and ramp-filtering |
| 1403 | Combining the partial images to generate a reconstructed image |

**F I G. 14**

1501 — Obtaining a plurality of temporally disconnected projection data sets of a object

1502 — Recording a physiologic signal from the scanned object

1503 — Determining levels of contributions for the obtained data sets by modifying their size or weighting their data

1504 — Reconstructing the image using obtained data and the determined levels of contribution

F I G. 15

F I G. 16